# EUROPEAN PATENT SPECIFICATION

(11) **EP 1 645 245 B1**
(45) Date of publication and mention of the grant of the patent: **27.07.2011**
(21) Application number: 04736986.3
(22) Date of filing: 17.06.2004
(51) Int. Cl.: A61F 2/06, A61B 17/11

(54) **INTRACARDIAC DEVICE WITH SEALABLE FENESTRATION FOR TOTAL CAVOPULMONARY ANASTOMOSIS BY CATHETERISATION**
INTRAKARDIALE VORRICHTUNG MIT VERSCHLIESSBAREM FENSTER FÜR EINE KAVOPULMONALE TOTALANASTOMOSE DURCH KATHETERISIERUNG
DISPOSITIF INTRACARDIAQUE POURVU D'UN FENETRAGE POUVANT ETRE OBTURE ET DESTINE A REALISER UNE ANASTOMOSE CAVOPULMONAIRE TOTALE PAR CATHETERISME

(30) Priority: 18.06.2003 AR 0302162; 08.09.2003 AR 0303251
(43) Date of publication of application: 12.04.2006
(73) Proprietor: Gamboa, Ricardo, Tolosa (La Plata), Buenos Aires (AR); Azcona Gamboa, Martin, 28027 Madrid (ES)
(72) Inventor: GAMBOA, Ricardo, Tolosa (La Plata), B-os. Aires (AR)
(74) Representative: Riera Blanco, Juan Carlos
(86) International application number: PCT/ES2004/000281
(87) International publication number: WO 2004/110314

(56) References cited:
- EP-A2- 1 312 320
- WO-A1-02/26310
- WO-A2-02/24108
- US-A- 5 445 600
- US-A- 5 653 743
- US-A1- 2003 130 719
- US-B1- 6 562 066

## Description

The present invention has the main object of an asymmetric intracardiac device to complete total cavopulmonary anastomosis by catherisation.

The secondary object of the present invention is a particular fenestration, selectively applicable to a portion of said asymmetric intracardiac device which can be closed and opened by catherisation when the operating cardiologist so desires.

### Field of application of the present invention:

The present invention is applicable in paediatric cardiology, more specifically in interventional haemodynamics for the correction of certain congenital heart diseases.

### Prior art - Single-ventricle physiology and treatment:

There are different congenital heart diseases (hereinafter "CHD"), which have just one functional ventricle, and this situation makes it necessary to establish a therapeutic strategy that enables a special haemodynamic model to be developed.

These situations are found in babies born with this heart malformation, and its correction by appropriate surgical interventions is essential.

To this effect, different surgical techniques have been devised in the last few decades, tending to sequentially prepare the circulatory apparatus, with the final aim of connecting the venous blood which returns to the heart by the inferior and superior vena cavas with the pulmonary circuit, so the blood is oxygenated.

This involves creating a bridge to the right ventricle, due to the fact that in these CHD this structure, which may be non-existent or rudimentary, is incapable of performing its function of lift pump for the blood towards the pulmonary circulation. Its objective is to maintain blood flow through the smaller circuit, due to the suction function of the single active ventricle, which is the left ventricle. Said circuit should have low flow resistance, without obstruction sites, so that the blood circulates suitably although it is not pumped in a pulsatile manner.

The following table shows the different CHD that merit surgical correction:

### Heart diseases that require a single-ventricular physiology:

● Single ventricle
● Atresia tricuspidea
● Left heart hypoplasia
● Pulmonary atresia with intact septum and right ventricle hypoplasia;
● Translocation of large arteries with unrelated intraventricular connection and small right ventricle;
● Double LV exit with bad anatomy:
   ● Superoinferior heart with crossed AV connection (criss-cross heart);
   ● Genuine RV hypoplasia
   ● Ebstein's disease (according to its anatomic variant).

Most of these CHD require a sequential treatment sequence such as that described below. Some of them (such as left heart hyperplasia) need special treatment, whose last phase is common to all of them.

When the patient is born with a single ventricle (prototype CHD of this physiopathology) and pulmonary stenosis or atresia, which prevents a pulmonary flow to maintain correct oxygenation, a ***modified Blalock-Taussig*** anastomosis with a 4 mm prosthetic tube between the subclavian artery and the homolateral pulmonary branch, typically of the left side, should be performed early.

If there is no pulmonary stenosis, a few weeks after the patient is born, a pulmonary artery cerclage should be performed to narrow its opening and limit the flow and pressure transmitted to the pulmonary circuit. This prevents the development of pulmonary hypertension, which would exclude the patient from the possibility of advancing with the following stages.

In any of the situations described, when the patient is 6 to 8 months of age, a ***bidirectional Glenn shunt*** is performed, which consists of separating the superior vena cava (SVC) of the right auricle (RA) and connecting it with the right pulmonary branch. In this way, all the venous blood from the upper part of the body passes directly to the pulmonary artery (PA) for oxygenation without entering the heart. This is performed because at this age the head and the arms represent 55% of the venous return. This technique is performed open-heart with extracorporeal circulation.

The last step consists of completing the total cavopulmonary anastomosis at approximately 3 to 4 years of age, carrying the flow of the inferior vena cava (IVC) to the pulmonary artery; also under extracorporeal circulation (ECC). Surgical techniques have been substantially modified in recent decades, mainly at this stage. From the original ***Fontan-Kreutzer*** surgery, which consisted of connecting the right auricular appendix to the right pulmonary branch (atriopulmonary anastomosis), to the current anastomosis with extracardiac tube between the IVC and the PA, various techniques have been tested. The latter consists of anastomosing the IVC to the right pulmonary branch (RBP) with the interposition of a Gore-Tex extracardiac prosthetic tube, which has a fenestration or orifice to the RA as "discharge" to ensure the minute volume in the post-operative period. This step completes the so-called "total cavopulmonary anastomosis" (TCPA).

Lately, new attempts have been made to use a covered stent to complete this last stage through an intervention by catherisation, avoiding further surgery, simplifying the technique, minimising the risks and the side effects of the ECC. These stents consist of an expandable tubular mesh of different materials, such as a stainless steel wire mesh, a nickel-titanium mesh or platinum-iridium mesh and covered with an impermeable polymer, such as expanded polytetrafluoroethylene (PTFE). Said devices connect the IVC to the SVC, after surgical preparation in the ***bidirectional Glenn procedure.***

Both the TCPA surgery with the extracardiac tubes and the current stents have the drawback of providing an unbalanced flow to the pulmonary circulation. In the known stents there is one or several fenestrations that make possible the "discharge" of blood from the circuit if the haemodynamic condition is not optimum permitting a short-circuit from right to left (venoarterial) on an auricular level, to maintain the cardiac minute volume in the post-operative period. These openings or fenestrations need to then be closed or plugged when the haemodynamic condition of the patient so permits.

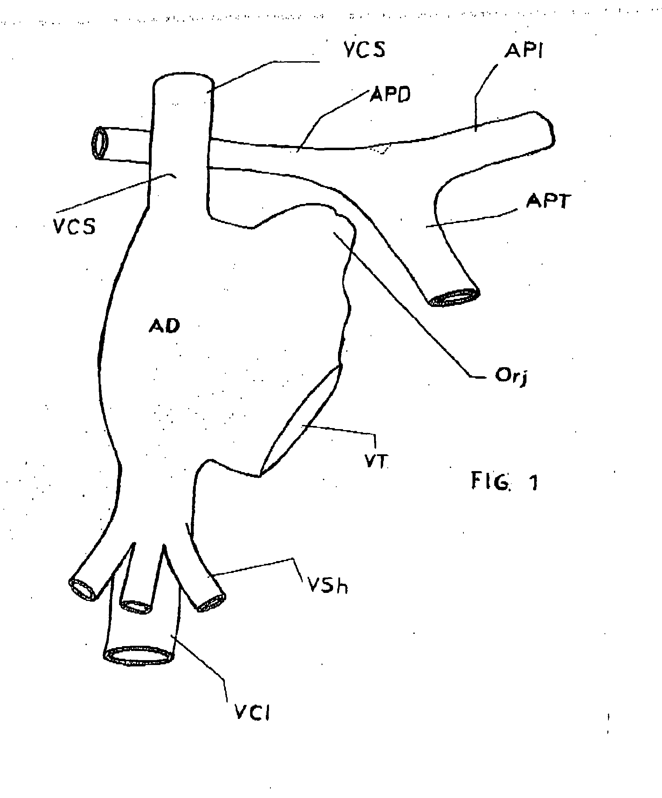

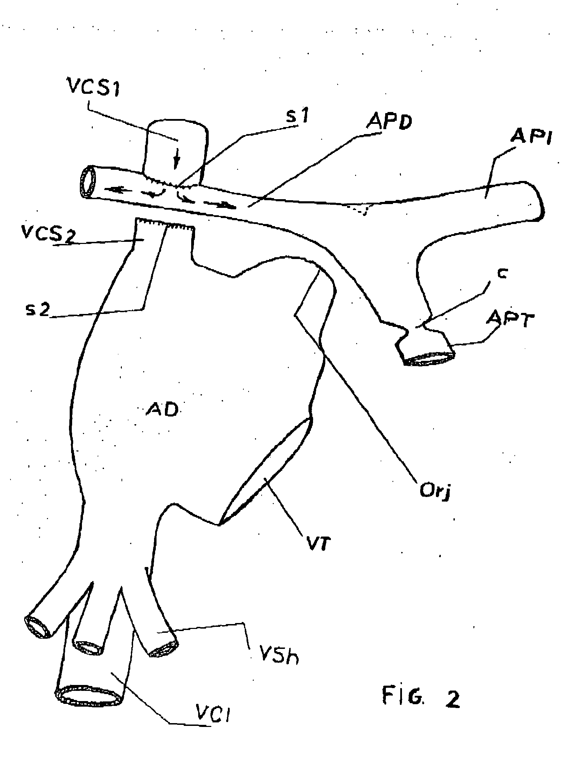

For the purposes of providing a suitable reference framework to the prior art of the present invention, Figure 1 schematically illustrates a heart which suffers from these CHD, in its state prior to the **Glenn's** procedure, and figure 2 illustrates typical **Glenn's** procedure to this same heart, of course in a schematic, simplified form and in accordance with the prior art.

The following notations have been used in these 2 figures:
● PA = Pulmonary Artery
● SVC = Superior Vena Cava
● IVC = Inferior Vena Cava
● SHV = Suprahepatic Veins
● App = Right auricular appendix
● RA = Right Auricle
● TV = Tricuspid valve

As references of these more modern known techniques and with the support of Figure 2, we can quote:

### ● SURGICAL PRECONDITIONING AND COMPLETION OF TOTAL CAVOPULMONARY CONNECTION BY INTERVENTIONAL CARDIAC CATHERIZATION: A NEW CONCEPT (Heart 1997; 75: 403-409).

This technique is used during the bidirectional Glenn surgery (Hemi-fontan procedure) to prepare the terrain to complete the total cavopulmonary connection by catherisation in high-risk patients.

A band or cerclage is left between the RA and the SVC, positioning a Gore-Tex tube with 3 to 7 perforations (multi-fenestrated) within the RA. In the future intervention, the cerclage is dilated with or without a Palmaz stent between the SVC-RA, and the fenestrations are sealed with 17 mm Rashkind devices, used to close the arterial conduit. If this is impossible, the positioning of a Gore-Tex tube in a covered stent is chosen.

### ● A NOVEL TECHNIQUE FOR ESTABLISHING TOTAL CAVOPULMONARY CONNECTION: FROM SURGICAL PRECONDITIONING TO INTERVENTION COMPLETION (J. Thorc Cardiovascular Surg 2000; 120; 1007-9).

This technique provides the experimental establishment of a cavo-caval anastomosis with a covered stent by a catherisation. A latero-terminal anastomosis is previously performed between the SVC and the RPA with PTFE. The SVC is connected to the RA with bands. In the following procedure, a stent graft is inserted endovascularly from the right internal jugular vein, positioning it by banding the SVC between the SVC-RPA and the IVC above the opening of the suprahepatic veins. This thus completes the cavo-caval-arteria pulmonary anastomosis.

### ● EFFECT OF BAFFLE FENESTRATION ON OUTCOME OF THE MODIFIED FONTAN OPERATION (Circulation 1992; 86; 1762-1769).

This technique demonstrates the benefits of the fenestration in Fontan operations in patients with increased risk. This study compares 91 patients who were left with a 4mm fenestration in the intracardiac tube with another 56 patients without fenestration. It is concluded that the fenestrated tube is associated with low mortality, lower incidence of pleural effusion with fewer days hospitalised.

To date, none of these interventions has given optimum results, a significant number of patients needing several further operations in the long term.

From approximately the age of six upwards, the systemic venous return percentage which is maintained to adulthood is attained. 35% of the pulmonary flow, in a normal adult without congenital heart disease, is provided by the SVC and the remaining 65% by the IVC. The right anatomically larger lung should receive approximately 55% of the blood flow and the smaller left lung, 45% of said flow, which involves a division of the flow from the IVC by 20% of the total flow (30.7% of the IVC flow) which should go to the RPA branch, whilst the remaining 45% should go to the LPA.

With the corrective techniques of CHD known to date, it is not always feasible to ensure a correct division of pulmonary blood flow, resulting in deficient supply depending on the technique used, of one lung or the other, typically the left one.

Another problem in the known techniques and devices, corrective of said CHD and which may involve serious drawbacks, is given by the fact that the cross-section of the IVC in older children has an average of 18 to 20 mm, whilst the PA has an approximate average diameter of 10 to 13 mm. The known techniques and devices resolve the problem by connecting the upper end of the extracardiac conduit of the PA by suture and flattening it, and this transforms a circular theoretical section in an elliptical theoretical cross-section, with the consequent reduction in the area, increasing resistance to the passage of the blood flow if it can be presumed to have a reasonably constant rate.

A final problem is found in the longitudinal dimensions if the device is intracardiac, as obviously not all the conformations of the patients affected have the same dimensions, and the device should adapt to somatic growth.

In turn, and providing the secondary object of the present invention, it is extremely useful to have the possibility that these intracardiac devices may, in a portion therein, have a selectively open or closed bypass or opening, according to the surgeon's criteria, called "fenestration" in the art, it then being possible to seal this fenestration at the cardiologist's criteria, without having to resort to open thoracic surgery.

Particularly, one of the possible fields of greater application of the secondary object of the present invention is found in Interventional Haemodynamics in congenital heart disease, and more specifically, in paediatric cardiology.

US-A-5 653 743 describes a hypogastric artery bifurcation graft designed to be placed in every hypogastric artery so as to maintain hypogastric flow while stenting the entire aorta-iliac system with endovascular overlapping straight tube grafts. This bifurcated device enables aorto-iliac stenting without colonic ischemic

### Object of the present invention:

A main object of the present invention is to achieve a device in the form of a covered stent or endoprosthesis to complete the total cavopulmonary correction or anastomosis via an intervention by cardiac catherisation. This device is applicable by means of a procedure to perform on the congenital heart diseases (CHD) which merit single-ventricle correction. Previously, an anatomical preparation should be performed during ***bidirectional Glenn*** surgery.

A further object of the present invention is an intracardiac device which makes possible a harmonic distribution of the blood flow dynamics, capable of contemporaneously by-passing between 30 to 35% of the blood flow from the IVC towards the right branch of the PA and between 65 to 70% of the blood flow to the left branch of the PA, substantially re-establishing a physiological blood flow distribution to both lungs, complemented with the flow which the preceding operation, the bi-directional Glenn shunt, preferably provides to the right lung.

A further object of the present invention is a covered stent-type device or endoprosthesis which permits interrupting the blood flow from the pulmonary artery trunk (in the event of cerclage thereof) or sealing the ***Blalock-Taussig anastomosis*** (in cases with pulmonary stenosis or atresia).

Another object of the invention is an intracardiac device whose cross-sections make it possible to compensate the change in shape (flattening of the cross-section) and to produce a reasonably constant cross-section.

Another object of the invention is an intracardiac device which permits adapting and compensating the dimensional differences existing in the RA of the various patients.

An object of the invention is a device which makes it possible to channel blood from the IVC to the PA in its connection between the trunk and the right pulmonary branch.

An object of the invention is a device which makes it physiologically possible to distribute the pulmonary flow, combined with a ***bidirectional Glenn*** shunt, improving the existing models.

An object of the invention is a device which permits treating the distortion of the pulmonary tree, reducing total resistance.

An object of the invention is a device which makes it possible to establish a blood flow with less kinetic energy loss with respect to the existing ones.

An object of the invention is a device which makes it possible to cover the somatic growth of the heart by its left convex curvature and permits re-expansions in its diameters.

An object of the invention is a device which permits bypassing blood from the liver (IVC) to both lungs, a physiologically important circumstance if we want to avoid pulmonary arteriovenous fistulas.

With respect to the secondary object of the present invention, it provides that in a portion of said device, open at both ends and interspersed in a body fluid circulatory system, this tubular body has at least one side opening or fenestration which defines a passage for the blood flow bypassed from the main circulatory flow passing through said tubular body, and this tubular body, in cooperation with said fenestration, incorporating intrinsic means for its selective sealing, without having to use any other additional plug element. In other words, the secondary object of the present invention is that the same tubular body carries the sealing means of the fenestration and that said sealing means that are always present do not alter the passage of body fluid nor its circulatory system.

Another secondary object of the present invention is that said tubular body consists of a conduit segment with a fenestration made in a portion of its side wall, the interior of said conduit segment having intrinsic selective-sealing means of said opening actionable by catherisation, avoiding a new operation, minimising the risks of said intervention and its unwanted side effects.

An object of the invention is a device which makes it possible to discharge the blood through the fenestration incorporated towards the RA in those cases that are not ideal ("High Risk Patients").

Finally, a secondary object of the present invention is that if, in the cardiologist's criterion, the already sealed fenestration should be reopened, said aperture and its subsequent closure can be performed by actuating the intrinsic sealing means positioned inside the segment of the tubular body by means of respective catherisations.

### Brief description of the invention:

SELECTIVELY FENESTRATED ASYMMETRIC INTRACARDIAC DEVICE TO COMPLETE TOTAL CAVOPULMONARY ANASTOMOSIS BY CATHERISATION, as defined in claim 1.

The secondary object of the invention provides that said first lower tubular portion may have at least one fenestration with intrinsic selective-sealing means, which defines a by-pass of the flow of a body fluid passing through the tubular body to the exterior thereof. Said fenestration is made in a portion of wall of the tubular body which defines a first arched surface of protruding convexity, elongated and non-deformable under the normal demands of exercise, with its major axis positioned according to a shunt of said side wall and arranged between both ends of the tubular body; this first arched surface determines a substantially oval shape in its intersection with the side surface of the tubular body from which it projects and said fenestration is made in the lower end of said first arched surface, downstream considering the forward direction of the body fluid flow throughout the tubular body; this first arched surface and said fenestration internally cover the tubular body by a second arched surface positioned between the interior of the tubular body and the first arched surface, both the first and the second arched surfaces being connected to the same oval perimeter in the tubular body, and both arched surfaces opposite one another have the same development and identical cross-sections; this second arched surface has an opening made within the same oval perimeter which connects the interior of the tubular body with the space defined between both arched surfaces, but positioned at the end opposite that of the fenestration made in said first arched surface, and positioned upstream with respect to the forward direction of the body fluid within the tubular body; the first arched surface projects out of the side of the tubular body and is fixed, whilst the oval perimeter connection defined between both surfaces defines a hinged area for the second arched surface with respect to the first one, this second arched surface being elastically deformable around said perimeter, retaining two stable operating positions with elastic recovery of its former shape; in the first of said operating positions, the second arched surface projects its convexity towards the inside of the tubular body, acquiring a convexity opposed to that of the first arched surface, establishing a passage for the flow of body fluids between both arched surfaces, which connect to said fenestration, all the cross sections of both arched surfaces thus being opposed equally and of contrary type; whilst in the second position said arched surface acquires a convexity of equal type to that of the first arched surface and rests against the interior surface of said surface determining the closure of the passage between both surfaces by a seal relation and sealing said fenestration and said opening in their respective surfaces; the interior of the second surface having a means of fastening for a device capable of displacing said second dorsum between both said operating positions; said tubular body and both arched surfaces being made in a material impermeable to the passage of body fluid.

### Preferred examples of embodiment of the invention:

For the purposes of exemplifying the preferred embodiments of the present invention, the following drawings are attached which illustrate this, with the support of the description thereof given at the beginning; this example of embodiment should be interpreted as one of the many possible constructions of the invention, for which reason no limiting value should be applied thereto, being included within the scope of protection thereof the equivalent possible means to those illustrated; the scope of the present invention being determined by the first claim attached in the corresponding claims section.

Likewise, these figures identify identical and/or equivalent means.
- Figure 1: shows, schematically, the part of a heart with said congenital heart disease, only showing the area of influence relative to its RA;
- Figure 2: illustrates the same portion of the heart as in Figure 1, wherein a ***Glenn*** shunt has been performed.
- Figure 3: shows a side elevational view of one of the possible device constructions of the invention;
- Figure 4: shows a close-up of a constructive variant of the device;
- Figure 5: shows the device of Figure 3, rotated 90° around its axis;
- Figure 6: schematically illustrates the relation of areas in the cross-sections with the projections of different sections and illustrates the concept of the division of the blood flow that rises through the IVC in the fork of the device of the invention;
- Figure 7: illustrates the device of the invention housed within the heart of figure 2;
- Figure 8: shows cut AA of Figure 3, and
- Figure 9: illustrates cut BB of Figure 3.
- Figure 10: shows a top view of an embodiment of a secondary object of the invention, consisting of a fenestration device, selectively-sealable by catherisation, this figure showing a portion of the lower tubular conduit of the stent of Figure 3, with the selective-sealing means of the invention seen from the outside, and in the foreground.
- Figure 11: shows a longitudinal cut of the embodiment of Figure 10 wherein the sealing device in its upper part, seen from outside, is observed.
- Figure 12: illustrates an exploded constructive detail of one of the possible constructions for the embodiment of Figures 10 and 11.
- Figure 13: illustrates another embodiment of the invention consisting of a stent with the sealing means of the invention seen from the outside and in the foreground.;
- Figure 14: shows a perspective view of a longitudinal cut of the embodiment of figure 12, wherein the inside part of the sealing device is observed, seen, from outside;
- Figure 15: permits viewing the device of figure 10, seen schematically in a longitudinal diametric cut, with the sealing means in their open position, i.e. with the passage of blood flow open.
- Figure 16: shows the same representation of Figure 15, illustrating the sealing means in their closed position, i.e. sealing the passage of blood flow;

Figure 1, as has been mentioned, schematically illustrates the RA of a heart with a heart disease typical of those already mentioned, in its state prior to the ***Glenn*** shunt;

Figure 2 shows the same RA, with the ***Glenn*** shunt already performed, which basically consists of sectioning the SVC, suturing the upper portion SVC1 to the RPA branch by connection s1, whilst the SVC2 that connected the RA is sutured and closed with suture s2. A cerclage or restriction in the "c" passage in the TPA has previously been performed.

With this intervention, the heart is prepared for the subsequent aforementioned interventions.

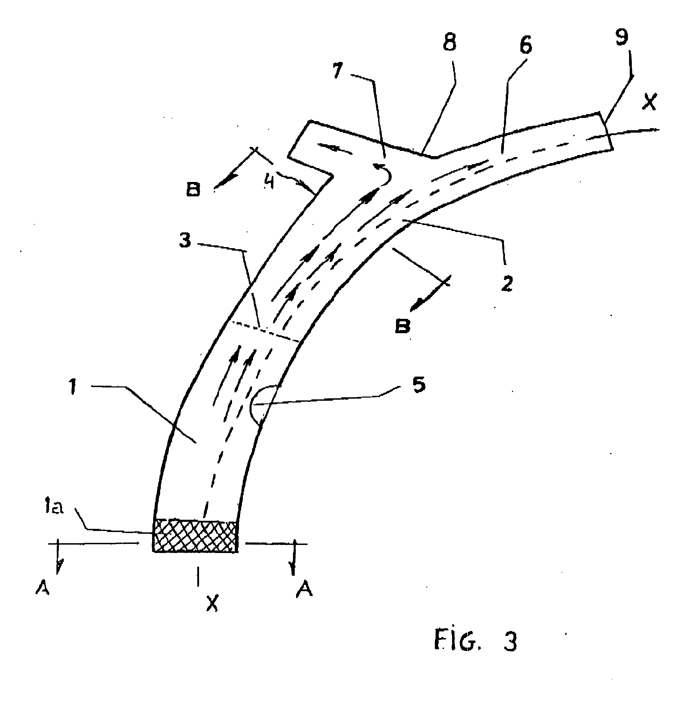

The invention includes an asymmetric intracardiac device that defines a covered stent or endoprosthesis, seen in Figure 3, comprised of a first lower portion (1) and a second upper portion (2). Both portions (1,2) follow one on from the other according to the same deformed X-X axis, arranged in the space forming a single tubular conduit.

The first lower portion (1) is formed by a mesh totally similar to a mesh component of a stent, i.e. a mesh preferably made from joined metal wire, (linked or woven) in its intercrossing, and coated with an impermeable polymeric material, such as polytetrafluoroethylene (PTFE). The lower part (1a) of the lower portion (1) is preferably not coated and is inserted in the IVC, permitting the non-coated mesh portion to collect blood from the ShV.

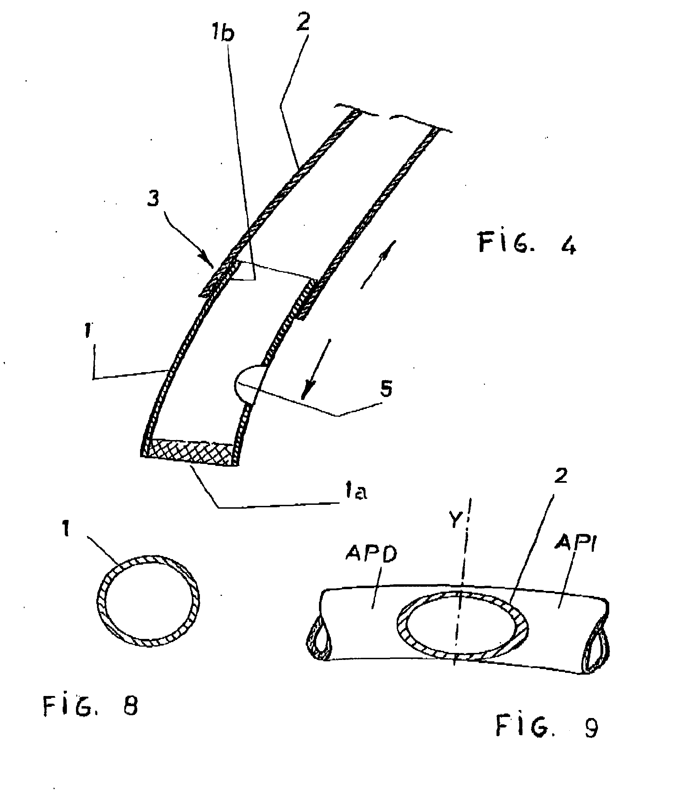

There may be two different embodiments, with respect to the two portions (1, 2). In one of said constructions, the lower portion (1) is assembled or inserted axially within the second upper portion (2), the reference (3) indicating the connection zone, this detail being displayed in Figure 4. This construction permits the interventional cardiologist to establish a telescopic adjustment of the total length of the device and adapt it to the anatomy of each patient, displacing the portion (1b) which is inserted in the lower end (2a) of the upper portion (2).

The other possible embodiment is that wherein the lower portion (1) follows on from the upper portion (2) forming a single piece.

From the point of view of the material comprising it, both portions (1, 2) of the device may be comprised of the same mesh or the lower portion (1) can be produced by a more rigid mesh and with a different weft, and the upper portion (2) comprised of a softer, more flexible mesh. We would like to emphasize therefore that the device of the invention, in its single-body version, or instead two telescopically axial bodies, may have the same mesh of equal resistance throughout the device, or the comprising portions may have a mesh of varied rigidity, production and elasticity.

The first portion (1) comprises a curvature between 35° and 45°, having at its lower end (1a) a substantially circular cross-section, with a diameter between 16 and 20 mm, as shown in Figure 8, which shows cut AA of Figure 3, whilst the adjacent area of the Y-fork and the second portion has a progressively flattened cross-section and is substantially oval in shape, illustrated in Figure 9, which shows cut BB of Figure 3.

One of the important conditions of the invention, in one of its preferred embodiments, is that the cross-sections throughout said XX axis have substantially equal area from the lower end (1a) until reaching the area (4) immediately downstream from the fork detailed below. Precisely, as the device of the invention has to join or be linked in this zone with the TPA in its connection to the RPA branch, whose average diameter is 12 mm, then the device of the invention should have an oval or elliptical section there whose smaller diameter according to axis Y of Figure 9 is equivalent to approximately 12 mm, which permits supporting the major axis in coincidence with the longitudinal axis of the RPA, consequently managing to maintain a cross-section of equal area.

The side of said first portion (1) has at least one fenestration (5) whose diameter may be an average of 4 mm, which connects the interior of said tubular conduit to the exterior thereof.

After this second portion (2) reaches a height equivalent to the section indicated with (4) in Figure 3, this second portion (2) forks into two branches. One of said branches is longer and has substantially circular cross-sections with diameter between 10 to 13 mm and following on from said deformed XX axis and is indicated with reference (6), tightly being inserted in the LPA, being hermetically adjusted to the internal walls thereof and plugging the entrance to the TPA.

The other branch (7) is projected in the form of a short appendix of substantially circular section, with a diameter of 10 to 13 mm and obliquely divergent with subsequent inclination, forming a deformed "Y" with respect to the longer branch, this short branch (7) being inserted in the beginning of the RPA.

In the device, the length of the first portion (1) ranges from 60 to 75 mm, whilst the longer branch (6) of the second portion has a length between 18 and 25 mm, and the length of the short forked appendix ranges from 4 to 8 mm.

Another fundamental aspect of the invention is that it provides a balanced flow distribution to the RPA and LPA according to a physiological model. For this reason, the device of the invention provides that the longest branch (6) follows on from the deformed XX axis, but after said fork, the cross-section of (6) is substantially circular, with a diameter around 12 mm. As it comes from an elliptical tubular conduit (4) with an area equivalent to a circle with an average diameter of 18 mm, the cross-area of (6) is markedly less than the cross section of (2) in area (4), so that the short appendix (7) which precisely starts in this area (4) is projected from a cross-area equivalent to a circumference with an average diameter of 18 mm. The transition between these two cross-areas is overcome as it has an arm (7) of a wall (9) substantially perpendicular to the blood flow that rises through (1,2), forcing a part of the flow to bypass through (7) on hitting against (8).

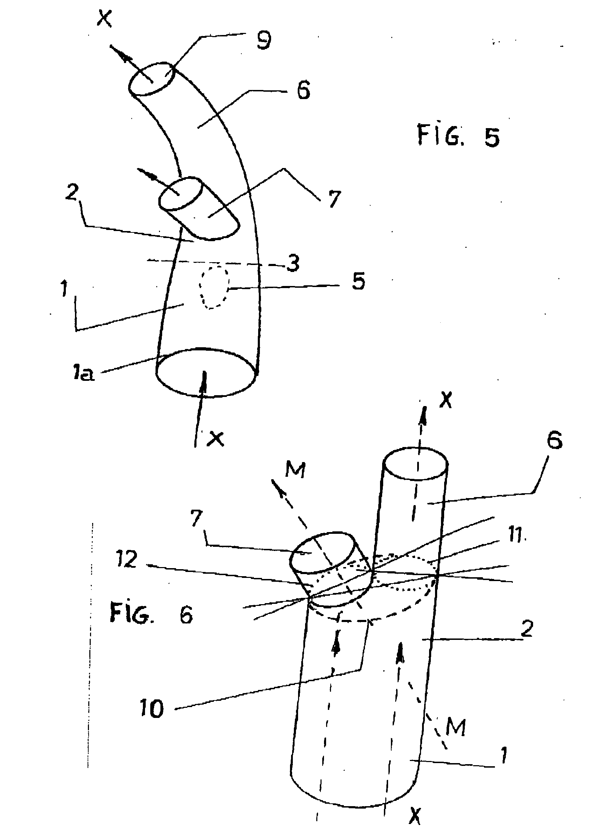

In other words, the short appendix (7) in its fork with respect to the longer branch (6) defines a portion of wall (8) which opposes between 50% and 70% of the projected area that traverses the blood flow that rises through the tubular conduit (1, 2) from its lower end, as indicated by the arrows in Figures 3 and 6. This short appendix (7) may be coated, or may simply consist of a mesh, without coating.

Another embodiment of the invention provides that the branch (6) of the fork has a slightly decreasing cross-section towards its free end, to be used in cases wherein one wants to limit the blood flow towards the LPA, and increase the flow towards the RPA by a small percentage, at the criteria of the interventional cardiologist.

Figure 5 shows the device of Figure 3, projected in side elevation from its left side. It is highlighted that the XX axis is deformed in space, and the end (9) of the branch (6) is directed backwards as is branch (7). It is also observed in the figure that the cross-sections of the portion (2) are flattened until attaining a smaller diameter compatible with the diameter of the LPA.

Figure 6 is an idealisation that shows the area relations between the various branches of the device, showing the component portions in a slight side perspective, as if they had straight lines and constant circular cross-sections. This simulation makes it possible to establish that the XX axis, as it is aligned with portion (6) of the fork, is displaced to one side of the device, whereas branch (7) of the same fork is aligned with another axis, MM. These two axes are representative of the blood flows to separate and circulate via (6) and (7). In said fork, it is interesting to note that portion (2) has a cross-area (10), with cross-areas (11) and (12) respectively belonging to portions (6) and (7). It is observed that areas (11, 12) are smaller than area (10) in a proportion substantially coinciding with the flow percentage by-passed from the corresponding branch (6) or (7).

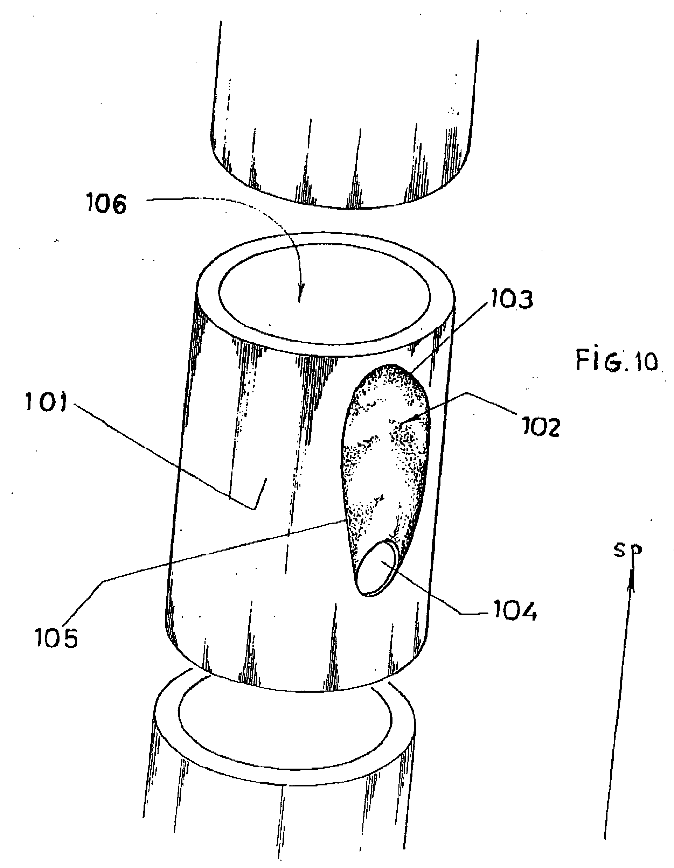

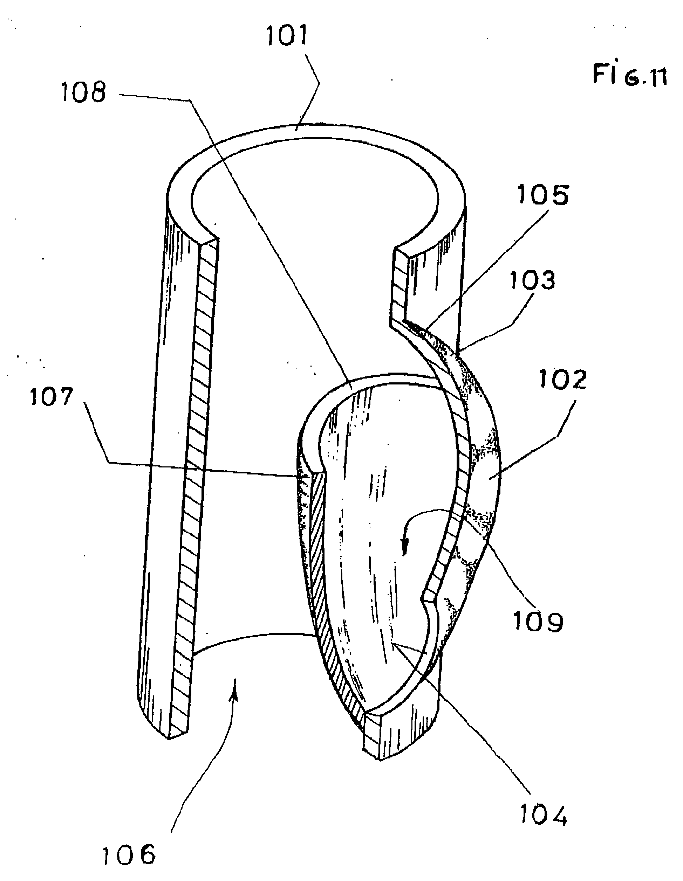

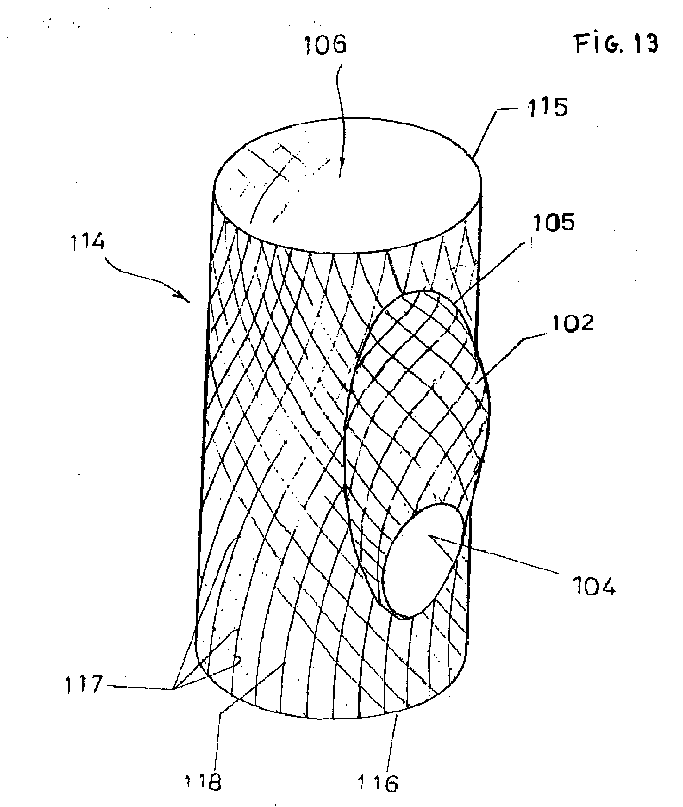

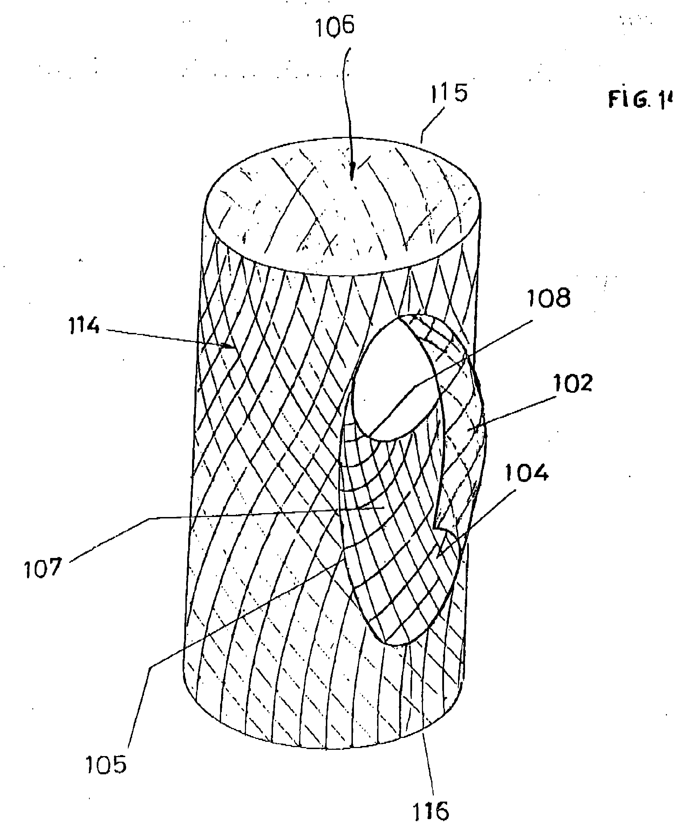

If we now consider the secondary aspect of the present invention, i.e. the selectively-sealable fenestration, we see in Figure 3 that the device of the invention has a fenestration. This fenestration (5) is preferably provided with selective-sealing means, actionable by the interventional surgeon by means of catherisation, without having to resort to open surgery. Figures 10 to 16 only display the lower portion (1) of the stent of Figure 3, in the portion including the fenestration (5).

On the side (101) where this is performed there is at least one window or fenestration (5) for the blood flow from the interior (106) of the tubular mesh towards the exterior thereof and this fenestration is made in a portion of tubular mesh wall that defines an elongated recess (107), and with its convexity defining a first arched dorsum or longitudinal axis substantially parallel to the longitudinal axis of said tubular portion.

In other words, each fenestration (5) performed on the side of the stent defining a first convex arched surface (107) and preferably this fenestration (5) is positioned on the lower end of said first convex arched surface (107) determining an opening (108). This first arched surface may have various forms depending on design requirements, but it is preferred that it has the shape of an elongated tear, and with its perimeter (105) pointed or irregular elliptical.

This perimeter (105) is marked on the side (101) of the tubular mesh from where the deformation of the convex arched surface (107) commences.

This first arched surface (107) together with said fenestration (5) whose opening is now (108), is covered by a second metal wire mesh walls also coated with a polymer impermeable to the passage of blood and which determines a second arched surface (102) with a convexity equal and opposed to that of the first arched surface (107), this second arched surface (102) being projected towards the exterior of the tubular conduit, whilst (107) is projected towards the interior thereof. Both first and second arched surfaces (102, 107) are connected to a first perimeter (105) of the tubular mesh body, and both projections that are mutually opposite have the same development and identical cross-sections.

The second arched surface (102) remains fixed with respect to the tubular body (101), whilst the first arched surface (107) may rotate around its perimeter (105) which defines a hinge line, making use of its characteristics of elastic flexibility and it assumes two stable operating positions with elastic recovery of its former shape, as this portion of the first arched surface (107) is elastically deformable around said perimeter. In the first of said operating positions, the first arched surface (107) projects its convexity to the interior (106) of the tubular mesh conduit (101), acquiring a convexity of equal section and opposite type to that of the second arched surface (102).

As has already been mentioned, the window or fenestration of opening (108) is made in the first arched surface (107) upstream from the blood circulation according to the position of the stent (see "sp" arrow), whilst the second arched surface (102) has an opening (104) at its opposite end to that of the fenestration (108) made in said first arched surface (107). This opening (104) is open towards the outside of the tubular body (101).

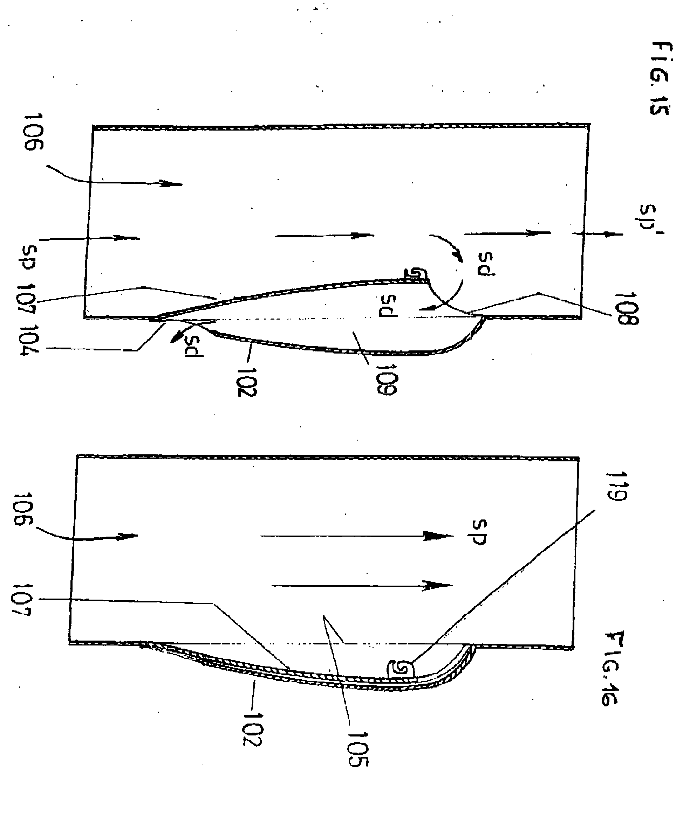

In said first operating position, the first arched surface (107) has its convexity projecting towards the interior of the stent, and as it is specularly opposite the second projection (102) it establishes the passage (109) for the bypassed blood flow between both. In consequence, in this first operating position, the blood flow (sp) which moves forwards through the interior of the tubular conduit, on being opposite the window or fenestration (108,) is partially by-passed in (sd), penetrating through the window (108) to the passage (109) and from there it exits the stent through the window (104) of the second arched surface (102) and is bypassed towards the point determined by the cardiologist. The blood flow exiting through the end upstream is (sp'), with sp' = sp - sd). (See figure 15).

The exact shape of the curves of both arched surfaces (102, 107) depends on the design criteria of each stent for each particular disease, as the are of the window and the outlet (104) determining the by-passed blood flow and also the shapes of the convexities are critical to avoid whorls, turbulences and back-flow.

In said figures, it is observed that the entrance of the window (108) preferably has an upwards-increasing elliptical leading edge, to avoid turbulences an the creation of vacuums in the leading edges. Likewise, the outlet profile (104) is also elliptical.

In the second operating position (see figure 16) said first arched surface (107) is pushed and elastically deformed, the mesh portions rotating as units on the perimeter (105), until attaining a convexity of equal type to that of the second arched surface (102), wherein it recovers its original shape and rests against the interior surface of said second arched surface (102), fitting against this and establishing a double wall with a large contact surface, determining the sealing of the passage (109) between both arched surfaces with a sealing relation and sealing said fenestration (108) and opening (104) in the second arched surface (102). This is possible because as the shapes of both are coincident arched surfaces, the fenestration of window (108) is sealed by the deformed mesh (107) on elastically recovering and fitting against the internal wall of (102) and similarly, the bottom of (107) fits onto the bottom of the interior face of (102) and seals the outlet (104), It is further observed that the second arched surface (102) has a slight bulge (103) in the areas adjacent to its end upstream opposite (108) and this is due to the need to ensure the abutting of both surfaces in close contact with one another to establish a good sealing relation.

One of the main advantages of the present invention lies in its capacity to seal the bypass passage (109) and selectively promote its release, re-establishing the passage of blood flow via (109), with the recourse of displacing the first elastic arched surface (107) between both aforementioned positions. For this reason, in part of the interior surface of (107), and arranged within the tubular space (106) of the stent, there are hooking means, such as a hook (119) which defines hooking means for a device capable of displacing to said first arched surface between its two operating positions, such as a catheter (not illustrated) which, when it passes through the corresponding vein, on lifting the position of said hook (119), pushes or pulls it depending on the case, closing or opening the passage (109) respectively, without need for an actual surgical intervention.

Finally, another advantage that is by no means less important is that this fenestration may be opened and/or closed by catheterisation as many times as the cardiologist considers necessary.

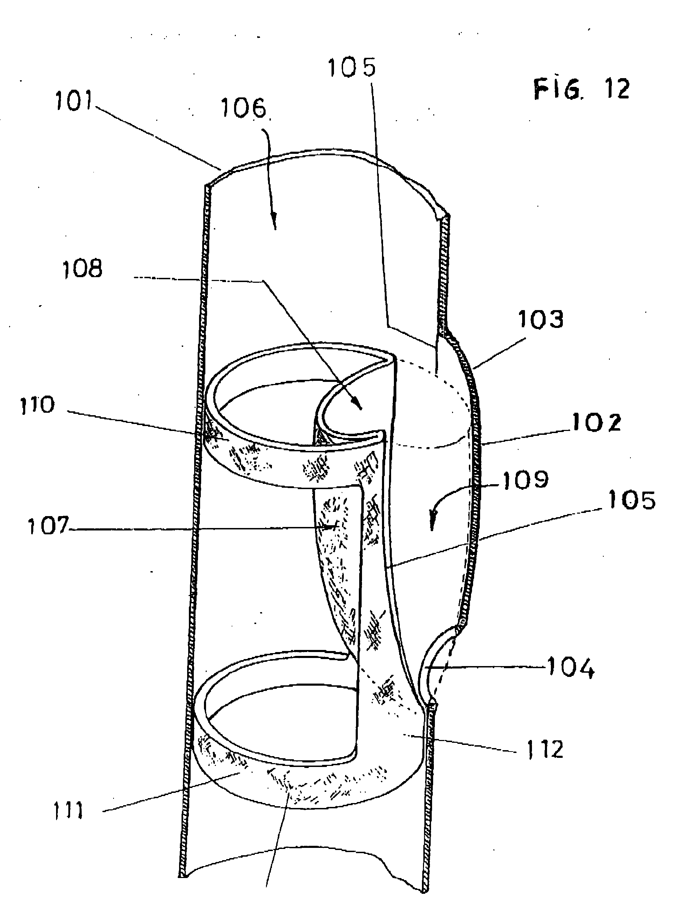

Figure 12 shows another construction for this selectively-sealable fenestration. The figure shows a construction which permits solving the problem caused when the polymer of the conduit (101) or tube is very soft, leading to the flattening of the tube in the critical area of both arched surfaces. Indeed, if said area was flattened, the volume the second arched surface (102) occupies in its projections towards the interior of the tube may even cause its occlusion, with the logical problems derived therefrom.

Figure 12 displays one of the possible solutions to said problem consisting of a construction wherein the second arched surface (107) is formed by a piece in a more rigid material which is inserted within the PTFE tube, which is joined to two annular portions (110, 111) which rest against the corresponding portions of the internal wall of the tube (101), these annular portions being connected to a cylindrical surfaced or half-round portion (112), from which a recess is formed therein, at the second arched surface (107), defining an intersection of said surface with said half-round (112) to the perimeter (105) which determines the oval hinge around which the second arched surface pivots and elastically deforms (7). The piece illustrated in Figure 12 conveniently is a monolithic piece, internally welded to the inner wall of (101). Said Figure 12 represents said reinforced part by a plurality of metal or textile fibres (113) incorporated in its mass, whilst in all embodiments of the present invention, the arched portion (102) is conveniently produced by a localized deformation of the wall (101).

### Comments regarding the technique of application of the present invention:

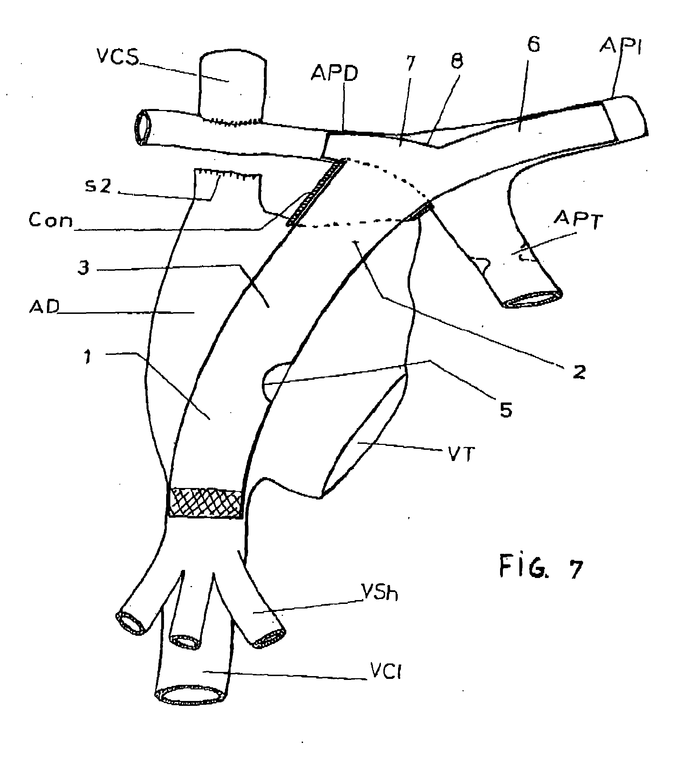

Figure 7 shows the device of the invention positioned within the RA, according to one of many possible techniques, without this technique being neither unique nor exclusive.

This technique consists of joining the end of the appendix (App) with the neck of the TPA to the RPA oriented towards the LPA. This connection can be made by suturing the end of the (App) and then piercing from inside the RA to let the fork (6, 7) of the device pass through, or to suture a short conduit (Con) connected to the (App) with said root of the TPA, piercing or cutting and passing the portion of the device through said conduit (see figure 7).

The connection site is in the surgically preestablished connection between the right appendix (App) and the right pulmonary artery (RPA) close to the pulmonary trunk TPA. This connection avoids the sinusal nodule and the complication caused by conduit disturbances. The surgical technique, during the previous ***Glenn*** shunt, may provide a reinforcement with Gore-Tex anastomosing both anatomic references and fitting the upper surface of the right App with the lower one of the proximal right branch. The "floor" of the right branch will freely open and the "arch" of the App will be cross-sectioned with an elliptical area and subsequently the incisions will be sutured leaving an opaque radial phantom in its central part.

Likewise the simple surgical suture between the Apρ and the RPA will enable the subsequent piercing with a transeptal needle to gain the position of the PA from the RA and the device to advance within a release system.

The device, in its unit version, is self-expandable, being released on removing the case or wrapping that contains it. When it is unfolded from its distal end (LPA) it will be positioned observing the anatomical reservations until the forking site. The mechanisms whereby the arm (7) of the device or right branch of the fork is released is by elastic recovery of its shape and direction, due to the fact that it is perpendicularly telescoped within the tubular body of the device. The primary anchoring is produced when both branches are intubated. The secondary anchoring is at IVC level.

In its two-body version, the upper or distal portion (2) is self-expandable and is inserted in the pulmonary branches as described above. The lower (1) or proximal portion which contains the fenestration may be produced in more rigid or less flexible material, and may be balloon-expanded, being inserted with in the upper portion (2).

### List of references

Figure 1:
   VCS - SVC
   APD - RPA
   API - LPA
   APT - TPA
   Orj - App
   VSh - ShV
   VCI - IVC
Figure 2:
   VCS1 - SVC1 VCS2 - SVC2 APD - RPA API - LPA APT - TPA Orj - App VT - TV VSh - ShV VCI - IVC
   Figure 7:
   VCS - SIC
   APD - LPA
   API - LPA
   VT - TV
   VSh - ShV
   VCI - IVC
   AD - RA
   CON - CON

## Claims

1. FENESTRATED ASYMMETRIC INTRACARDIAC DEVICE TO COMPLETE TOTAL CAVOPULMONARY ANASTOMOSIS BY CATHERISATION, which device comprises a forked tubular conduit (1,6,7), wherein said forked tubular conduit comprises a first lower portion (1) and a second upper portion (2), both portions following on from the other one according to the same axis (XX) deformed in space forming a tubular conduit portion; the first portion (1) comprises a tubular mesh coated at least partly thereof by an impermeable polymer, with a curvature between 35° and 45°, said first portion having a substantially circular cross-section at its lower end (1a), with a diameter between 16 and 20 mm, whilst at its upper end said portion has a cross-section whose successive cross-sections are progressively flattened and with substantially oval shape, the cross-sections throughout said axis being substantially of equal area; the side of said first portion has at least one selectively-sealable fenestration (5, 104) which connects the inside of said tubular conduit to the outside thereof; this first lower portion (1) is continued by said second upper portion (2) which comprises a tubular mesh at least partially coated by an impermeable polymeric material and with cross-sections along said deformed axis (XX), these cross-sections becoming oval or elliptical with a smaller diameter of approximately 12 mm, the cross-sections being substantially of equal area; after the second portion obtains said diameter, it forks into two branches (6, 7), one (6) of said branches being of greater length and substantially circular cross-sections of diameter between 10 and 13 mm and following on said deformed axis, whilst the other branch (7) is projected in the form of a short appendix of substantially circular cross-section, with a diameter of 10 to 13 mm and obliquely divergent forming a deformed "Y" with respect to the longer branch, with its oblique, divergent branches directed backwards from the longer branch; each one of said branches is formed by a mesh comprised of wires at least partially coated by said impermeable polymeric material and forms a single body with respect to the second upper portion; the length of the first portion (1) being between 60 and 75 mm, whilst the longer branch (6) of the second portion (2) has a length of between 18 and 25 mm, and the length of the short forked appendix (7) ranging from 4 to 8 mm; the short appendix (7) defining in its fork, with respect to the longer branch, a portion of wall which confronts between 50 and 70% of the projected area that traverses the blood flow that rises through the tubular conduit from its lower end; the lower end of the first portion (1) defines a connection to the inferior vena cava and the suprahepatic veins; this tubular conduit formed by said first and second portions (1, 2) is formed to be housed within the right auricle of the heart, whilst the longer branch (6) of the fork is to be housed within the left pulmonary artery, establishing a relation of close contact with the internal walls thereof and defining an obstruction with respect to the pulmonary arterial trunk, whilst the short appendix (7) is to be housed in the beginning of the right pulmonary artery.

2. FENESTRATED ASYMMETRIC INTRACARDIAC DEVICE, according to claim 1, **characterized in that** the first lower portion (1) and the second upper portion (2) form a single tubular body produced by at least a series of wires arranged to form a mesh.

3. FENESTRATED ASYMMETRIC INTRACARDIAC DEVICE, according to claim 1, **characterized in that** the first lower portion (1) is comprised of an independent mesh portion of the second upper portion (2), said first portion (1) being axially displaceable and positionable within the second portion (2), defining a tubular body of selectively variable length.

4. FENESTRATED ASYMMETRIC INTRACARDIAC DEVICE, according to claims 1 to 3, **characterized in that** the first lower portion (1) has a mesh with filaments of greater resistance than the filaments that form the second upper portion (2), determining a first lower portion of less flexibility with respect to the second upper portion.

5. FENESTRATED ASYMMETRIC INTRACARDIAC DEVICE, according to claims 1 to 4, **characterized in that** the lower end (1a) of this first portion (1) has a mesh structure without polymeric coating defining a tubular conduit end permeable to the passage of blood which rises through the inferior cava vein and suprahepatic veins.

6. FENESTRATED ASYMMETRIC INTRACARDIAC DEVICE, according to claim 1, **characterized in that** the branch of greater length (6) of said fork is formed from a mesh comprised of wires totally coated by said impermeable polymeric material, forming a continuity of the tubular wall impermeable to the passage of blood with said second upper portion, whilst the other branch (7) of said fork is not coated by said impermeable material, or only partially coated forming a short appendix permeable to the passage of blood.

7. FENESTRATED ASYMMETRIC INTRACARDIAC DEVICE, according to claims 1 to 6, **characterized in that** the mesh material that defines the portions thereof is formed by thin metal wires connected in their intercrossing, forming a deformable mesh and with sufficient memory to acquire its original shape and dimensions once released from the deforming action; the impermeable polymeric material being polytetrafluoroethylene (PTFE) or similar.

8. FENESTRATED ASYMMETRIC INTRACARDIAC DEVICE, according to claims 1 to 7, on whose side there is at least one through-hole (5, 104) which defines a fenestration to by-pass a flow of a body fluid passing from the interior of the tubular body to the exterior thereof, **characterized in that** said fenestration (5, 104) is made in a portion of wall of the tubular body which defines a first arched convex surface (107) with convexity projected into the inside from said portion of wall, this first surface defining an elongated shell, non-deformable under normal exercise requirements, with its major axis positions according to a shunt of said side wall and arranged between both ends of the tubular body; this first arched surface (107) defining, in its intersection with the side surface of the tubular body from which it projects, a perimeter of substantially oval shape and said fenestration (5, 104) is made at the lower end of said arched surface, downstream considering the forward direction of the body fluid flow throughout the tubular body; this first arched surface (107) and said fenestration (5, 104) are internally coated on the tubular body by a second arched surface (102) positions
between the interior of the tubular body and the first arched surface, both first and second arched surfaces (107, 102) being only connected together by the same oval perimeter in the tubular body, both mutually opposite arched surfaces having the same development and identical cross-sections; this second arched surface (102) has an opening (104) made in the same oval perimeter, which connects the interior of the tubular body with the space (109) defined between both arched surfaces, but positioned at its end opposite that of the fenestration made in said first arched surface (107), and positioned downstream from the forward direction of the body fluid within the tubular body; the first arched surface projects outwards fro the side of the tubular body and remains fixed and non-deformable, whilst the oval perimeter connection defined between both surfaces defines a hinged area for the second arched surface (102) with respect to the first (107), this second arched surface being elastically deformable around said perimeter (108), retaining two stable operating positions with elastic recovery of its previous shape wherein, in the first of said operating positions, the second arched surface (102) projects its convexity towards the outside of the tubular body, acquiring a convexity of opposite type to that of the first arched surface (107), establishing a passage for the flow of body fluids between both arched surfaces, the interior of the tubular body connecting with said passage via said opening, which connects said fenestration (104), with all the cross-sections of both arches surfaces thus equally and oppositely confronting one another; whilst in the second position, said second arched surface (102) acquires a convexity of equal type to that of the first surface (107) and rests against the interior surface of said first surface determining
the closure of the passage between both surfaces with a seal relation and sealing said fenestration and said opening on their respective surfaces; the interior of the second surface (102) having a means capable of displacing said second arched surface between its two said operating positions; said tubular body and both arched surfaces being made in a material impermeable to the passage of the body fluid.

9. FENESTRATED ASYMMETRIC INTRACARDIAC DEVICE, according to claim 8, **characterized in that** said tube is soft and the portion thereof that defines the first arched surface (107) has an elastic resistance to deformation, of greater size than the rest of the tube, said first arched surface (107) resting on a substantially non-deformable carrier structure (112) and which rests on the inside of said tube, in correspondence with and faced to the concavity of said first arched surface.

10. FENESTRATED ASYMMETRIC INTRACARDIAC DEVICE, according to any of claims 8 to 9, **characterized in that** at least the first and second arched surfaces (107, 102) comprise a layer of polymeric material, wherein a mesh is positioned with this mesh being formed from filaments chosen from textile filaments and metal filaments.

11. FENESTRATED ASYMMETRIC INTRACARDIAC DEVICE, according to claim 8, **characterized in that** said tubular portion comprises a stent, defined by a filament or metal wire mesh coated by an impermeable polymeric material, the second arched surface being defined by another portion of filament or metal wire mesh coated with an impermeable polymeric material, with the whole defining elastically deformable walls. prior to be placed in a patient.

## Patentansprüche

1. FENESTRIERTE ASYMMETRISCHE INTRAKARDIALE VORRICHTUNG ZUR VOLLSTÄNDIGEN KAVOPULMONALEN ANASTOMOSE DURCH KATHETERISIERUNG, **dadurch gekennzeichnet, dass** diese Vorrichtung eine verzweigte röhren- bzw. schlauchförmige Leitung (1, 6, 7) umfasst, welche aus einem ersten unteren Abschnitt (1) und einem zweiten oberen Abschnitt (2) besteht, die beide an der gleichen Achse (xx) aufeinander folgen und unter räumlicher Verformung einen röhrenförmigen Leitungsabschnitt bilden, dessen erster Abschnitt (1) ein Geflecht aus Netzschlauch umfasst, das zumindest teilweise mit einem undurchlässigen Polymer beschichtet ist und eine Krümmung zwischen 35° und 45° besitzt, wobei der besagte erste Abschnitt (1) an seinem unteren Ende (1a) einen im Wesentlichen runden Querschnitt eines Durchmessers zwischen 16 und 20 mm aufweist, während sich die Querschnittsflächen des genannten Abschnitts an seinem oberen Ende zunehmend abflachen und eine im Wesentlichen ovale Form annehmen, wobei die einzelnen Querschnittsflächeninhalte entlang der besagten Achse im Wesentlichen gleich sind, **dadurch gekennzeichnet, dass** an der Seite des genannten ersten Abschnittes mindestens eine wahlweise verschließbare Fenestrierung (5, 104) angebracht ist, welche die Innenseite der besagten röhrenförmigen Leitung mit ihrer Außenseite verbindet, wobei der erste untere Abschnitt (1) vom genannten zweiten oberen Abschnitt (2) fortgesetzt wird, der ein Geflecht aus Netzschlauch umfasst, das zumindest teilweise mit einem undurchlässigen Polymer beschichtet ist und dessen Querschnittsflächen entlang der genannten verformten Achse (xx) in eine ovale bzw. elliptische Form mit einem kleineren Durchmesser von etwa 12 mm übergehen, wobei diese Querschnittsflächeninhalte im Wesentlichen gleich sind, **dadurch gekennzeichnet, dass** der zweite Abschnitt, sobald er den genannten Durchmesser angenommen hat, sich in zwei Zweige (6, 7) aufteilt, wovon Zweig (6) der längere ist, im Wesentlichen runde Querschnittsflächen mit Durchmessern zwischen 10 mm und 13 mm besitzt und der besagten verformten Achse folgt, während der andere Zweig (7) in Form eines kurzen Fortsatzes im Wesentlichen runden Querschnitts eines Durchmessers von 10 bis 13 mm herausragt und auf schräg auseinander strebende Weise bezüglich des längeren Zweigs ein verformtes Y bildet, dessen schräg auseinander strebende Zweige vom längeren Zweig aus nach hinten gerichtet sind, **dadurch gekennzeichnet, dass** beide besagte Zweige aus einem Drahtnetz bestehen, das zumindest teilweise mit dem erwähnten undurchlässigen Polymer beschichtet ist, und hinsichtlich des zweiten oberen Abschnittes einen einzigen Körper bilden, wobei der erste Abschnitt (1) zwischen 60 und 75 mm, der längere Zweig (6) des zweiten Abschnittes 18 bis 25 mm und der verzweigte kurze Fortsatz (7) von 4 bis 8 mm lang ist, wobei Letzterer in seiner verzweigten Form bezüglich des längeren Zweiges einen Wandabschnitt bestimmt, der einem Anteil von 50 bis 70% der projizierten Fläche gegenüber liegt, welche vom Blut durchquert wird, das ausgehend vom unteren Ende durch die röhrenförmige Leitung nach oben strömt, **dadurch gekennzeichnet, dass** das untere Ende des ersten Abschnittes (1) eine Verbindung zur unteren Hohlvene und zu den suprahepatischen Venen herstellt, wobei diese röhrenförmige Leitung, die aus dem erwähnten ersten und dem zweiten Abschnitt (1, 2) besteht, für die Einführung in den rechten Herzvorhof bestimmt ist, während der längere Zweig (6) der Verzweigung in der linken Lungenarterie untergebracht wird, somit einen engen Kontakt mit den Innenwänden dieser Arterie herstellt und hinsichtlich des Stammes der Lungenarterien eine Verstopfung bildet, während der kurze Fortsatz (7) zur Unterbringung im Anfangsstück der rechten Lungenarterie bestimmt ist.

2. FENESTRIERTE ASYMMETRISCHE INTRAKARDIALE VORRICHTUNG nach Anspruch 1, **dadurch** ausgezeichnet, dass der erste untere Abschnitt (1) und der zweite obere Abschnitt (2) einen einzigen röhrenförmigen Körper bilden, der mindestens aus einer Reihe von Drähten besteht, die in Form eines Drahtnetzes angeordnet sind.

3. FENESTRIERTE ASYMMETRISCHE INTRAKARDIALE VORRICHTUNG nach Anspruch 1, **dadurch** ausgezeichnet, dass der erste untere Abschnitt (1) ein vom zweiten oberen Abschnitt (2) unabhängiges Netzwerk umfasst, wobei sich dieser erste Abschnitt (1) innerhalb des zweiten oberen Abschnitts (2) axial verschieben und positionieren lässt und auf diese Weise einen röhrenförmigen Körper wählbarer variabler Länge bildet.

4. FENESTRIERTE ASYMMETRISCHE INTRAKARDIALE VORRICHTUNG nach den Ansprüchen 1 bis 3, **dadurch** ausgezeichnet, dass die Fasern des ersten unteren Abschnittes (1) widerstandsfähiger sind als die Fasern des zweiten oberen Abschnittes (2), wodurch der erste untere Abschnitt weniger flexibel ist als der zweite obere Abschnitt.

5. FENESTRIERTE ASYMMETRISCHE INTRAKARDIALE VORRICHTUNG nach den Ansprüchen 1 bis 4, **dadurch** ausgezeichnet, dass die Netzstruktur am unteren Ende (1a) des ersten Abschnitts (1) keine Polymerbeschichtung umfasst und ein röhrenförmiges Leitungsende bildet, das für Blut durchlässig ist, welches durch die untere Hohlvene und die suprahepatischen Venen aufsteigt.

6. FENESTRIERTE ASYMMETRISCHE INTRAKARDIALE VORRICHTUNG nach Anspruch 1, **dadurch** ausgezeichnet, dass der längere Zweig (6) der verzweigten Struktur aus einem Drahtnetz gebildet wird, das gänzlich mit dem genannten undurchlässigen Polymermaterial beschichtet ist und so eine Fortsetzung der röhrenförmigen Wand bildet, die für den Blutfluss in den zweiten oberen Abschnitt undurchlässig ist, während der andere Zweig (7) der erwähnten verzweigten Struktur nicht oder nur teilweise mit dem besagten undurchlässigen Material beschichtet ist und auf diese Weise einen kurzen blutdurchlässigen Fortsatz bildet.

7. FENESTRIERTE ASYMMETRISCHE INTRAKARDIALE VORRICHTUNG nach den Ansprüchen 1 bis 6, **dadurch** ausgezeichnet, dass das Netzmaterial, welches die einzelnen Abschnitte bildet, aus dünnen Metalldrähten besteht, die an ihren Kreuzungsstellen verbunden sind und ein verformbares Gewebe bilden, dessen Gedächtnis hinreicht, um nach dem Nachlassen einer verformenden Einwirkung wieder die Originalform und -größe anzunehmen, wobei das undurchlässige Polymer Polytetrafluorethylen (PTFE) oder ein ähnliches Material ist.

8. FENESTRIERTE ASYMMETRISCHE INTRAKARDIALE VORRICHTUNG nach den Ansprüchen 1 bis 7, an deren Seite sich mindestens eine Durchlassöffnung (5, 104) befindet, die eine Fenestrierung zur Umleitung des Flusses eines Körperfluids bildet, das vom Innenraum des röhrenförmigen Körpers in seinen Außenraum strömt, **dadurch gekennzeichnet, dass** diese Fenestrierung (5, 104) in einem Wandabschnitt des röhrenförmigen Körpers vorgenommen ist, wobei dieser Wandabschnitt eine erste konvex gekrümmte Fläche (107) bildet, deren Krümmung vom besagten Wandabschnitt nach innen ragt, wobei die eben genannte erste Fläche unter normalen Einsatzanforderungen eine nicht verformbare Schale bildet, deren lange Achse gemäß einem Shunt (Kurzschlussverbindung) der erwähnten Seitenwand derart zwischen den beiden Enden des röhrenförmigen Körpers positioniert ist, dass die Schnittfläche der erwähnten ersten gekrümmten Fläche (107) beim Durchgang durch die Seitenfläche des röhrenförmigen Körpers, aus dem die Fläche (107) herausragt, im Wesentlichen oval geformt ist, wobei die Fenestrierung (5, 104) am unteren Ende der besagten gekrümmten Fläche angebracht ist, also stromabwärts bezüglich der Flussrichtung des Körperfluids durch den röhrenförmigen Körper, außerdem **dadurch gekennzeichnet, dass** sowohl die erste gekrümmte Fläche (107) als auch die erwähnte Fenestrierung (5, 104) innen am röhrenförmigen Körper mit einer zweiten gekrümmten Fläche (102) beschichtet ist, die zwischen der Innenseite des röhrenförmigen Körpers und der ersten gekrümmten Fläche angebracht ist, wobei die erste und die zweite gekrümmte Fläche (107, 102) nur durch die oval umrissene Fläche im röhrenförmigen Körper miteinander verbunden sind und diese beiden einander gegenüber liegenden gekrümmten Flächen gleiche Krümmungsverläufe und identische Querschnitte besitzen, **dadurch** ausgezeichnet, dass am gleichen oval umrissenen Flächenabschnitt der zweiten gekrümmten Fläche (102) eine Öffnung (104) eingelassen ist, die den Innenraum des röhrenförmigen Körpers mit dem Raum (109) zwischen den beiden gekrümmten Flächen verbindet und welche sich, bezüglich der in der besagten ersten gekrümmten Fläche (107) angebrachten Fenestrierung, am gegenüberliegenden Ende befindet und hinsichtlich der Flussrichtung des Körperfluids durch den röhrenförmigen Körper stromabwärts positioniert ist, dergestalt, dass die erste gekrümmte Fläche aus der Seite des röhrenförmigen Körpers nach außen herausragt und fest und nicht verformbar bleibt, während die durch den ovalen Umriss begrenzte Verbindung beider Flächen für die zweite gekrümmte Fläche (102) bezüglich der ersten gekrümmten Fläche (107) einen aufklappbaren Bereich bildet, wobei die besagte zweite gekrümmte Fläche um den genannten Umfang (108) herum elastisch verformbar ist und zwei stabile Operationspositionen einnimmt, in die sie auf elastische Weise zurückkehrt, wobei die konvexe Form der zweiten gekrümmten Fläche (102) in der ersten stabilen Position in den Außenraum des röhrenförmigen Körpers hinein ragt und diese zweite Fläche somit eine zur ersten gekrümmten Fläche (107) entgegengesetzte konvexe Form annimmt, womit der Durchgang für Körperfluide zwischen den beiden gekrümmten Flächen gegeben ist, während der Innenraum des röhrenförmigen Körpers durch die besagte Öffnung mit dem genannten Durchgang und der Fenestrierung (104) verbunden ist und alle Querschnittsflächen der beiden gekrümmten Flächen einander gleich sind und sich gegenüber liegen; andererseits **dadurch gekennzeichnet, dass** die zweite gekrümmte Fläche (102) in der zweiten stabilen Position die gleiche konvexe Form der ersten gekrümmten Fläche (107) annimmt, an der Innenfläche derselben ersten Fläche anliegt und somit den Durchgang zwischen beiden Flächen sowie die Fenestrierung und die Öffnungen an beiden Flächen verschließt, wobei der Innenraum der zweiten Fläche (102) über eine Vorrichtung zur Verschiebung der besagten zweiten gekrümmten Fläche zwischen ihren beiden Operationspositionen verfügt, **dadurch gekennzeichnet, dass** der erwähnte röhrenförmige Körper und beide gekrümmte Flächen aus einem für den Durchfluss der Körperfluide undurchlässigen Material hergestellt sind.

9. FENESTRIERTE ASYMMETRISCHE INTRAKARDIALE VORRICHTUNG nach Anspruch 8, **dadurch gekennzeichnet, dass** das besagte Röhrchen (der röhrenförmige Körper) weich ist und jener Abschnitt dieses Röhrchens, der die erste gekrümmte Fläche (107) bildet, einen größeren Widerstand gegen elastische Verformung aufweist als der Rest desselben Röhrchens, wobei die besagte erste gekrümmte Fläche (107) auf einer im Wesentlichen nicht verformbaren Trägerstruktur (112) im Inneren des besagten Röhrchens ruht, entsprechend der konkaven Form der genannten ersten gekrümmten Fläche und derselben gegenüber liegend.

10. FENESTRIERTE ASYMMETRISCHE INTRAKARDIALE VORRICHTUNG nach den Ansprüchen 8 oder 9, **dadurch gekennzeichnet, dass** zumindest die erste und die zweite gekrümmte Fläche (107, 102) eine Schicht aus einem Polymermaterial umfassen, in der ein aus Textil- und Metallfasern gebildetes Netz angebracht ist.

11. FENESTRIERTE ASYMMETRISCHE INTRAKARDIALE VORRICHTUNG nach Anspruch 8, **dadurch gekennzeichnet, dass** der erwähnte röhrenförmige Abschnitt eine Gefäßprothese (Stent) umfasst, die aus Metallfasern oder aus einem Metalldrahtnetz mit einer Beschichtung aus einem undurchlässigen Polymermaterial besteht, während die zweite gekrümmte Fläche von anderen Fasern oder einem anderen Netz aus Metalldrähten gebildet wird, die ebenso mit einem undurchlässigen Polymermaterial beschichtet sind, wobei diese Gesamtheit elastisch verformbare Wände zur Positionierung in einem Patienten bildet.

## Revendications

1. Dispositif intracardiaque fenêtré asymétrique pour compléter une anatosmose cavo-pulmonaire par cathétérisation, ledit dispositif comprend une conduite tubulaire bifurquée (1, 6, 7), dans lequel ladite conduite tubulaire bifurquée (1, 6, 7) comprend une première portion inférieure (1) et une deuxième portion supérieure (2), les deux portions étant l'une derrière l'autre selon le même axe (XX) déformé dans l'espace en formant une portion de conduite tubulaire; la première portion (1) comprend une maille tubulaire revêtue au moins partiellement de celle-ci par un polymère imperméable, avec une courbure allant de 35° à 45°, ladite première portion ayant une section transversale essentiellement circulaire à son extrémité inférieure (1a), avec un diamètre allant de 16 a 20 mm, tandis qu'à son extrémité supérieure ladite portion a une section transversale dont les sections transversales successives sont progressivement aplaties et ont une forme essentiellement ovale, les sections transversales sur tout ledit axe ayant essentiellement la même surface, le côté de ladite première portion a au moins une fenestration sélectivement obturable (5, 104) qui relie l'intérieur de ladite conduite tubulaire à l'extérieur de celle-ci; cette première portion inférieure (1) est continuée par ladite deuxième portion supérieure (2) qui comprend une maille tubulaire au moins partiellement revêtue par une matière polymérique imperméable et avec des sections transversales le long dudit axe déformé (XX), ces sections transversales devenant ovales ou elliptiques avec un diamètre moindre d'environ 12 mm, les sections transversales ayant essentiellement une surface identique; après que la deuxième portion obtient ledit diamètre, elle se bifurque en deux branches (6, 7), une (6) desdites branches étant de longueur plus grande et de sections transversales essentiellement circulaires de diamètre allant de 10 à 13 mm et suivant ledit axe déformé, tandis que l'autre branche (7) est projetée sous la forme d'un appendice court de section transversale essentiellement circulaire, avec un diamètre allant de 10 à 13 mm et divergeant obliquement en formant un "Y" déformé par rapport à la branche plus longue, avec ses branches divergentes obliques dirigées vers le bas à partir de la branche plus longue; chacune desdites branches est formée par une maille constituée de fils au moins partiellement revêtue par ladite matière polymérique imperméable et forme un seul corps par rapport à la deuxième portion supérieure; la longueur de la première portion (1) allant de 60 à 75 mm, tandis
que la branche plus longue (6) de la deuxième portion (2) a une longueur allant de 18 à 25 mm, et la longueur de l'appendice court bifurqué (7) allant de 4 à 8 mm; l'appendice court (7) définissant dans sa bifurcation, par rapport à la branche plus longue, une portion de paroi qui fait face à entre 50 et 70% de la surface projetée qui traverse le débit sanguin qui monte à travers la conduite tubulaire à partir de son extrémité inférieure; l'extrémité inférieure de la première portion (1) définit une connexion entre la veine cave inférieure et les veines suprahépatiques; cette conduite tubulaire formée par lesdites première et deuxième portions (1, 2) est formée pour être logée à l'intérieur de l'oreillette droite du coeur, tandis que la branche plus longue (6) de la bifurcation est destinée à être logée à l'intérieur de l'artère pulmonaire gauche, en établissant une relation de contact étroit avec les parois internes de celle-ci et en définissant une obstruction par rapport au tronc de l'artère pulmonaire, tandis que l'appendice court (7) est destinée à être logée au début de l'artère pulmonaire droite.

2. Dispositif intracardiaque fenêtré asymétrique, selon la revendication 1, **caractérisé en ce que** la première portion inférieure (1) et la deuxième portion supérieure (2) constituent un seul corps tubulaire produit par au moins une série de fils disposés pour former une maille.

3. Dispositif intracardiaque fenêtré asymétrique, selon la revendication 1, **caractérisé en ce que** la première portion (1) inclut une portion de maille indépendante de la deuxième portion supérieure (2), ladite première portion (1) étant axialement déplaçable et positionnable à l'intérieur de la deuxième portion (2), en définissant un corps tubulaire de longueur sélectivement variable.

4. Dispositif intracardiaque fenêtré asymétrique, selon les revendications 1 à 3, **caractérisé en ce que** la première portion inférieure (1) a une maille avec des filaments de résistance supérieure aux filaments qui constituent la deuxième portion supérieure (2), en déterminant une première portion inférieure de moindre flexibilité par rapport à la deuxième portion supérieure.

5. Dispositif intracardiaque fenêtré asymétrique, selon les revendications 1 à 4, **caractérisé en ce que** l'extrémité inférieure (1a) de cette première portion (1) a une structure de maille sans revêtement polymérique définissant une extrémité de conduite perméable au passage du sang qui monte à travers la veine cave inférieure et les veines suprahépatiques.

6. Dispositif intracardiaque fenêtré asymétrique, selon la revendication 1, **caractérisé en ce que** la branche de plus grande longueur (6) de ladite bifurcation est formée à partir d'une maille comprenant des fils complètement revêtus par ladite matière polymérique imperméable, en formant une suite de la paroi tubulaire imperméable au passage du sang avec ladite deuxième portion supérieure, tandis que l'autre branche (7) de ladite bifurcation n'est pas revêtue par ladite matière imperméable, ou n'est que partiellement revêtue en formant un appendice court perméable au passage du sang.

7. Dispositif intracardiaque fenêtré asymétrique, selon les revendications 1 à 6, **caractérisé en ce que** la matière de maille qui définit les portions de celui-ci est formée par des fils métalliques fins reliés à leur intersection en formant une maille déformable et avec une mémoire suffisante pour acquérir sa forme et ses dimensions originales une fois libérée de l'action déformante; la matière polymérique imperméable étant le polytétrafluoroéthylène (PTFE) ou similaire.

8. Dispositif intracardiaque fenêtré asymétrique, selon les revendications 1 à 7, sur le côté duquel il y a au moins un trou traversant (5, 104) qui définit une fenestration pour dériver un débit d'un fluide corporel passant de l'intérieur du corps tubulaire à l'extérieur de celui-ci, **caractérisé en ce que** ladite fenestration (5, 104) est réalisée en une portion de paroi du corps tubulaire qui définit une première surface convexe cambrée (107) avec une convexité projetée à l'intérieure de ladite portion de paroi, cette première surface définissant une coque allongée indéformable dans les conditions normales de service, avec son grand axe positionné selon une dérivation de ladite paroi latérale et disposé entre les deux extrémités du corps tubulaire; cette première surface cambrée (107) définissant, dans son intersection avec la surface latérale du corps tubulaire à partir duquel elle se projette, un périmètre de forme essentiellement ovale et ladite fenestration (5, 104) est réalisée à l'extrémité inférieure de ladite surface cambrée, en considérant la direction en aval le sens de circulation du fluide corporel à travers tout le corps tubulaire; cette première surface cambrée (107) et ladite fenestration (5, 104) sont revêtues intérieurement sur le corps tubulaire par une deuxième surface cambrée (102) positionnée entre l'intérieur du corps tubulaire et la première surface cambrée, aussi bien la première que la deuxième surfaces cambrées (107, 102) étant seulement reliées par le même périmètre ovale dans le corps tubulaire, les deux surfaces cambrées mutuellement opposées ayant le même développement et des sections transversales identiques; cette deuxième surface cambrée (102) a une ouverture (104) réalisée dans le même périmètre ovale, qui relie l'intérieur du corps tubulaire à l'espace (109) défini entre les deux surfaces cambrées, et positionnée à son extrémité opposée à celle de la fenestration réalisée dans ladite première surface cambrée (107), et positionnée en aval du sens de progression du fluide corporel à l'intérieur du corps tubulaire; la première surface cambrée se projette à l'extérieur du côté du corps tubulaire et demeure fixée et indéformable, tandis que la connexion du périmètre ovale définie entre les deux surfaces définit une zone articulée pour la deuxième surface cambrée (102) par rapport à la première (107), cette deuxième surface cambrée étant élastiquement déformable autour dudit périmètre (108), en retenant deux positions opérationnelles stables avec récupération élastique de sa forme antérieure dans laquelle, dans la première desdites positions opérationnelles, la deuxième surface cambrée (102) projette sa convexité vers l'extérieur du corps tubulaire, en acquérant une convexité de type opposé à celle de la première surface cambrée (107), en établissant un passage pour l'écoulement de fluides corporels entre les deux surfaces cambrées, l'intérieur du corps tubulaire se reliant audit passage à travers ladite ouverture, qui relie ladite fenestration (104), toutes les sections transversales des deux surfaces cambrées se faisant face ainsi également et de manière opposée; tandis que dans la deuxième position, ladite deuxième surface cambrée (102) acquit une convexité de type identique à celle de la première surface (107) et elle repose contre la surface intérieure de ladite première surface en déterminant la fermeture du passage entre les deux surfaces avec une relation d-obturation et qui obture ladite fenestration et ladite ouverture sur leurs surfaces respectives; l'intérieur de la deuxième surface (102) ayant un moyen apte à déplacer ladite deuxième surface cambrée entre ses deux dites positions opérationnelles; ledit corps tubulaire et les deux surfaces cambrées
étant réalisées en une matière imperméable au passage du fluide corporel.

9. Dispositif intracardiaque fenêtré asymétrique, selon la revendication 8, **caractérisé en ce que** ledit tube est souple et portion de celui-ci qui définit la première surface cambrée (107) a une résistance élastique à la déformation de plus grande dimension que le reste du tube, ladite première surface cambrée (107) reposant sur une structure portante essentiellement indéformable (112) et qui repose à l'intérieur dudit tube, en correspondance avec et en opposition à la concavité de ladite première surface cambrée.

10. Dispositif intracardiaque fenêtré asymétrique, selon l'une quelconque des revendications 8 a 9, **caractérisé en ce qu'**au moins la première et la deuxième surfaces cambrées (107, 102) comprennent une couche de matière polymérique, dans lequel est positionnée une maille, cette maille étant formée de filaments choisis parmi les filaments textiles et les filaments métalliques.

11. Dispositif intracardiaque fenêtré asymétrique, selon la revendication 8, **caractérisé en ce que** ladite portion tubulaire comprend un "stent", défini par une maille de filaments ou fils métalliques revêtue par une matière polymérique imperméable, la deuxième surface cambrée étant définie par une autre portion de maille de filaments ou fils métalliques revêtue par une matière polymérique imperméable, le tout définissant des parois élastiquement déformable avant sa mise en place dans un patient.
